Europäisches Patentamt

European Patent Office

Office européen des brevets

(19)

(11) Publication number : **0 668 082 A1**

(12) **EUROPEAN PATENT APPLICATION**

(21) Application number : **95300480.1**

(22) Date of filing : **26.01.95**

(51) Int. Cl.⁶ : **A61L 27/00, C23C 14/02, C23C 14/06**

(30) Priority : **10.02.94 GB 9402625**

(43) Date of publication of application :
**23.08.95 Bulletin 95/34**

(84) Designated Contracting States :
**BE CH DE ES FR IT LI NL**

(71) Applicant : **UNITED KINGDOM ATOMIC ENERGY AUTHORITY**
**Harwell**
**Didcot, Oxfordshire OX11 0RA (GB)**

(72) Inventor : **Bull, Stephen John**
**55 Sinodun Road**
**Didcot, Oxfordshire OX11 8HW (GB)**
Inventor : **McCabe, Andrew Robert**
**5 Nursery Close**
**Headington, Oxford OX3 7AG (GB)**
Inventor : **Jones, Adrian Michael**
**9 Beaufort Close**
**Didcot, Oxfordshire OX11 8T (GB)**

(74) Representative : **Wood, Paul Austin et al**
**United Kingdom Atomic Energy Authority,**
**Patents Department 329,**
**Harwell**
**Didcot, Oxfordshire OX11 0RA (GB)**

(54) **Improved load-bearing polymeric materials.**

(57) A method of improving the tribological properties of a polymeric material, including the operations of subjecting the polymeric material to bombardment with energetic radiation such as to cause disruption of the polymeric chains and subsequent cross-linking thereby initially to harden the surface of the polymeric material and subsequently forming a layer of hard carbonaceous material on the hardened surface of the polymeric material.

Fig.2.

EP 0 668 082 A1

The present invention relates to polymeric materials the tribological properties of which have been enhanced, and more specifically to the improvement of the tribological properties of polymeric components of prostheses such as replacement hip and knee joints.

It is known that both the electrical and mechanical properties of polymeric materials can be altered by bombarding the polymeric materials with energetic ions of various light elements such as hydrogen, carbon, nitrogen and oxygen and heavy elements such as argon. The main structural modification processes which occur are the disruption of the polymer chains and cross-linking between them. The relative rates of each process will determine whether the properties of the material will be enhanced or degraded. Materials such as polytetrafluoroethylene are degraded by ion bombardment whereas others, such as polyimides, can have their load-bearing properties improved considerably.

For example in US patent specification 5,133,757 there is disclosed a process for improving the surface characteristics of orthopaedic implants made of plastics materials and, in particular, those made of ultra high molecular weight polyethylene (UHMWPE). In the process disclosed in the above specification, such implants are subjected to bombardment with ions of a material selected from the group comprising aluminium, magnesium, silicon, titanium, argon, helium, neon, nitrogen, fluorine, and chlorine. The ions are implanted with energies in the range 20 to 400 KeV and ion doses in the range $10^{13}$ - $10^{16}$ ions/cm$^2$ are used, giving atomic concentrations of the implanted ions of between 0.1 and 2 per cent.

The improvement in the surface properties of the implants is said to result from the densification of the near surface region of the plastics material due to the presence of increased carbon-carbon bonding in that region, the carbon-carbon bonding being diamond-like.

It is to be noted that in the above-disclosed process, no separate layer of diamond like carbon as such is formed. The result of the process is stated to be a densified layer, which may include diamond-like inter-carbon bonding, but also contains a concentration of foreign species, that is to say, species which are neither hydrogen nor carbon and which make their own, unacknowledged, contribution to the properties of the surface of the plastics implant.

It is known also that hard carbon coatings can be produced on a variety of materials by cracking hydrocarbons which have been deposited on the surfaces of the materials. A suitable method for doing this is disclosed in GB patent specification 2,122,224B.

It is an object of the present invention in one aspect to provide a method of improving the tribological properties of a polymeric material, and in another aspect to provide a polymeric material of enhanced tribological properties.

According to the invention in one aspect there is provided a method of improving the tribological properties of a load bearing surface of a body made of a polymeric material, including the operations of initially subjecting the body of polymeric material to bombardment with energetic radiation such as to cause disruption of polymeric chains within the polymeric material and subsequent cross-linking thereby initially to harden the surface region of the polymeric material and subsequently forming on the hardened surface region of the body of polymeric material, a layer of hard, generally amorphous, carbonaceous material having predominantly diamond type inter-carbon bonding or a mixture of diamond type and graphitic bonding.

Preferably the energetic radiation is in the form of ions having energies greater than 10 KeV.

Preferably the layer of hard carbonaceous material is formed by providing in the region of the said surface of the body of polymeric material a population of a species including hydrocarbon groups and subjecting the surface of the body of polymeric material to bombardment by energetic ions thereby to rupture the hydrocarbon bonds in the hydrocarbon groups in the said species and deposit the layer of hard carbonaceous material on the said surface of the body of polymeric material.

Alternatively, the layer of hard carbonaceous material can be formed by depositing from the vapour phase a layer of a hydrocarbon upon the said surface of the body of polymeric material and subjecting the layer of hydrocarbon to bombardment by energetic ions, thereby to rupture the hydrocarbon bonds and form the layer of hard carbonaceous material on the said surface of the body of polymeric material.

A preferred polymeric material is ultra high molecular weight polyethylene (UHMWPE). Suitable ions for the ion bombardment stage are those of hydrogen, nitrogen, argon, carbon, methane or acetylene with energies greater than 10 KeV and ion doses greater than $10^{13}$ per sq cm. Suitable ions for the formation of the hard carbonaceous layer by ion assisted coating are those of hydrogen, nitrogen, argon or methane.

Also according to the invention there is provided an artefact made of a polymeric material, including a load-bearing surface having a sub-surface region the hardness of which has been increased as a result of irradiation induced disruption and cross-linking of the polymer chains within the polymeric material and a layer of diamond-like carbonaceous material formed upon the hardened sub-surface region of the said surface of the artefact.

The artefact may be a load-bearing component of a prosthesis such as an acetabular cup for use in a replacement hip joint.

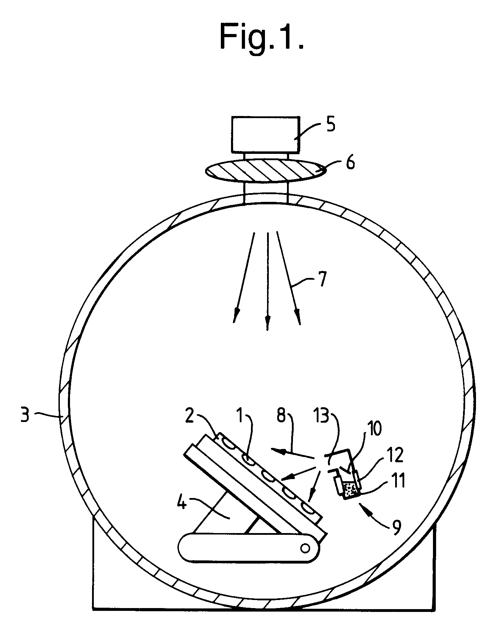

The invention will now be described by way of example, with reference to the accompanying drawing in which Figure 1 illustrates diagrammatically, a process according to the invention for treating the bearing surface of an acetabular cup, which forms part of a hip prosthesis, and

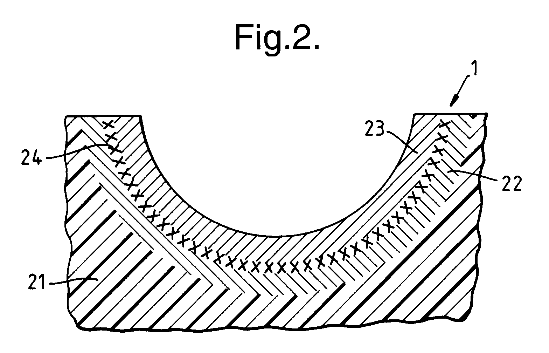

Figure 2 shows diagrammatically a cross-section of a portion of the acetabular cup of Figure 1.

A hip prosthesis consists of a femoral component which mimics the shape of the upper end of a femur and is inserted into the upper end of a patient's femur and an acetabular cup which is inserted into the patient's pelvic girdle. Normally the femoral component is made of a relatively biocidally inert metal such as a titanium alloy and the acetabular cup is made of a polymeric material with acceptable tribological properties such as UHMWPE. However, in use, transfer of the material of the acetabular cup to the femoral component occurs, with a consequent increase in the friction between the two components of the prosthesis, and a consequent increase in the rate of wear of the prosthesis. This, of course is undesirable, both because of the trauma associated with major operations and because the replacement of a hip joint is an expensive operation. Hence anything which prolongs the useful life of a replacement hip is highly desirable.

The present invention is particularly useful for extending the lifetime of a hip prosthesis.

Referring to the drawing, a number of acetabular cups 1 made of UHMWPE are placed in a holder 2 within a vacuum chamber 3. The holder 2 is arranged to be rotated by an electric motor 4. A volume ion source 5 and an electrostatic focusing lens 6 is arranged to produce a beam 7 of hydrogen ions. Also within the vacuum chamber 3 is a source of hydrocarbon vapour designed to produce a beam of vapour 8 at an angle of about 45° to the surface of the acetabular cup holder 2. The source of hydrocarbon vapour consists of an open can 9 which can be closed with a shutter 10 containing a quantity of vacuum pump oil 11, and a heater 12. The open end of the can 9 forms a nozzle 13 which directs the vapour towards the acetabular cups 1.

In one process embodying the invention, the acetabular cups 1 are cleaned using alcohol and deionised water, placed in position in the holder 2, the vacuum chamber 3 is evacuated to a vacuum of about $10^{-5}$ torr, the shutter 10 is closed, and the acetabular cups 1 are subjected to bombardment by a beam 7 of $H^+$ ions from the source 5 at an energy of about 50 KeV until an ion dose of between $10^{16}$ and $10^{17}$ ions per sq cm has been implanted. The heater 12 is then energised and the oil 11 is heated to a temperature of 150°C, so as to evaporate some of the oil 11 and produce a partial pressure of about $10^{-4}$ torr of oil vapour in the closed can 9.

Once the pre-bombardment of the cups is complete the shutter 10 is opened so as to enable the beam 8 of hydrocarbon vapour to be directed by the nozzle 11 to the region of the vacuum chamber 3 in which the acetabular cups 1 are situated. The acetabular cups 1 are again bombarded with $H^+$ ions in the presence of the oil vapour at an energy of about 50 KeV for a period of about four hours.

During this period the hydrocarbons in the oil vapour are cracked into carbon and hydrogen which by the mechanism of ion-assisted coating forms a layer of diamond-like carbon on the surfaces of the acetabular cups 1 which are exposed to the beam 7 of ions from the ion source 5. In addition some carbon atoms are recoil-implanted into the acetabular cups 1 and increase the disruption of the polymer chains of the UHMWPE out of which the acetabular cups are made and so assist crosslinking and the further hardening of the sub-surface regions of the acetabular cups 1.

Vacuum pump oils are not necessarily hydrocarbons, for example, one oil which often is used is polyphenyl ether. However, this material does contain hydrocarbon groups which if it is used as the source of carbon for deposition, are cracked in the same way as true hydrocarbon materials.

Acetabular cups treated according to the above process have been subjected to wear tests by sliding them against cobalt chrome femoral implant heads in the presence of saline solution at 37°C with loading according to the Paul walking cycle. The improvement in the wear rate compared with that of untreated acetabular cups made of UHMWPE is considerable. For example, in one experiment, after two million cycles, a wear depth of 0.1 mm for a treated acetabular cup was recorded, as opposed to a wear depth of 0.8 mm for an untreated acetabular cup.

The hardness of the diamond-like carbon surface layer on treated acetabular cups can be improved further by sterilisation techniques such as irradiating them with $\gamma$-radiation. In addition to sterilising the irradiated surfaces, a 10% increase in the hardness of the surface region of the acetabular cups is achieved.

Other ions and bombardment conditions which have been used for the initial stage of the process are:

| Ion | Energie (KeV) | Current Density (mA/Cm²) | Dose Range (ions/cm²) |
|---|---|---|---|
| $N_2^+$ | 50 | 0.62 | $3 \times 10^{15}$ - $1 \times 10^{16}$ |
| $N_2^+$ | 50 | 0.87 | $4 \times 10^{13}$ - $1 \times 10^{17}$ |
| $N_2^+$ | 50 | 0.87 | $2.5 \times 10^{15}$ - $1 \times 10^{17}$ |
| $Ar^+$ | 50 | 0.87 | $2.5 \times 10^{15}$ - $1 \times 10^{17}$ |
| $CH_4^+$ | 50 | 0.87 | $2.5 \times 10^{15}$ - $1 \times 10^{17}$ |
| $C_2H_2^+$ | 50 | 0.87 | $1 \times 10^{17}$ |

Other ions and bombardment conditions which have been used for the hard carbonaceous layer deposition stage of the process are:

| Ion | Energy (KeV) | Current Density ($mA/cm^2$) |
|---|---|---|
| $N_2^+$ | 50 | 0.87 |
| $Ar^+$ | 50 | 0.87 |
| $CH_4^+$ | 50 | 0.87 |

The above different ions were obtained by changing the gas fed to the ion source 5.

Ion energies in the range 10 KeV to 100 KeV, or even higher, can be used, together with ion doses up to $10^{18}$ ions/cm².

The apparatus described uses a volume ion source. However, if desired other forms of ion source such as a twin anode ion source can be used. Also, although it is convenient so to do, it is not necessary to use the same ions for both stages of the process.

Figure 2 shows diagrammatically a load-bearing portion 21 of an acetabular cup 1 treated by a process according to the invention. The portion 21 of the acetabular cup has a surface region 22 within the original part of the acetabular cup 1 in which considerable bond rupture and cross-linking has occurred so as to harden it, upon which there is deposited a layer 23 of diamond-like carbon. Between the two is an interfacial region 24 in which additional disruption of the polymer chains in the UHMWPE occurs, which both further hardens the UHMWPE and integrates the diamond-like carbon layer 23 with the main body of the portion 21 of the acetabular cup 1.

## Claims

1. A method of improving the tribological properties of a load bearing surface of a body made of a polymeric material, including the operations of initially subjecting the body (1) of polymeric material to bombardment with energetic radiation such as to cause disruption of polymeric chains within the polymeric material and subsequent cross-linking thereby initially to harden the surface region (22) of the polymeric material and subsequently forming on the hardened surface region (22) of the body (1) of polymeric material a layer (23) of hard carbonaceous material having predominantly diamond type inter-carbon bonding or a mixture of diamond type and graphitic bonding.

2. A method according to Claim 1 wherein the energetic radiation comprises ions having an energy greater than 10 KeV.

3. A method according to Claim 2 wherein the initial ion bombardment is continued until at least $10^{13}$ ions/cm² have been implanted into the polymeric material.

4. A method according to Claim 2 or Claim 3 wherein the ions are selected from the group comprising hydrogen, nitrogen, carbon, argon, methane and acetylene.

5. A method according to any of Claims 1 to 4 wherein the layer of hard carbonaceous material is deposited by means of an ion-assisted coating technique.

6. A method according to Claim 5 wherein the ions used to deposit the layer (23) of hard carbonaceous material are selected from the group comprising hydrogen, nitrogen, carbon and methane.

7. A method according to Claim 5 or Claim 6 wherein the ions used to deposit the layer (23) of hard carbonaceous material are the same as those used initially to harden the said surface region (22) of the body (1) of polymeric material.

8. A method according to Claim 5, Claim 6 or Claim 7 wherein the layer (23) of hard carbonaceous material is formed by establishing in the region of the surface (22) of the body (1) of polymeric material the tribological properties of which are to be improved a population of a species including hydrocarbon groups and subjecting the said surface (22) of the body (1) of polymeric material to bombardment with ions having an energy greater than 10 KeV thereby to disrupt the hydrocarbon groups in the said species and deposit the layer (23) of hard carbonaceous material upon the said surface (22) of the body (1) of polymeric material.

9. A method according to Claim 8 wherein the species including hydrocarbon groups is a carbonaceous oil vapour.

10. A method according to any preceding claim wherein there is included the operation of exposing the said surface (22) of the body (1) of polymeric material to $\gamma$-radiation after the layer (23) of hard carbonaceous material has been deposited upon it.

11. A method according to Claim 1 for improving the tribological properties of a surface of an artefact made of a polymeric material, wherein there is included the operations of chemically cleaning the said surface of the artefact (1), placing the artefact (1) in a vacuum chamber (3), evacuating the chamber (3) to a pressure in the region of $10^{-5}$ torr, subjecting the said surface of the artefact (1) to bombardment with ions having an energy of about 50 KeV until at least $10^{13}$ ions/cm$^2$ have been implanted into the said surface of the artefact (1), providing a population of a hydrocarbon containing species in the region of the said surface of the artefact at a partial pressure of the order of $10^{-4}$ torr and continuing the ion bombardment of the artefact (1) until a layer (23) of hard carbonaceous material of a desired thickness has been deposited upon the said surface of the artefact (1).

12. A method according to Claim 11 wherein the population of hydrocarbon species in the region of the said surface of the artefact is obtained by directing a stream of vapour (8) of a carbonaceous oil at the said surface of the artefact (1).

13. A method according to Claim 12 wherein the carbonaceous oil is polyphenyl ether.

14. A method according to Claim 11, Claim 12 or Claim 13 wherein the ions used both initially to harden the said surface of the artefact (1) and to deposit the layer (23) of hard carbonaceous material are hydrogen ions.

15. A method according to any of Claims 11 to 14 wherein the ion bombardment initially to harden the said surface of the artefact (1) is continued until an ion dose of up to $10^{17}$ ions/cm$^2$ has been implanted into the said surface of the artefact (1).

16. A method according to any of Claims 11 to 15 wherein the second ion bombardment is continued for a period of about 4 hours.

17. An artefact made of a polymeric material, wherein there is included a load-bearing surface having a sub-surface region (22) the hardness of which has been increased as a result of irradiation induced disruption and cross-linking of the polymer chains within the polymeric material and a layer (23) of diamond-like carbonaceous material formed upon the hardened sub-surface region of the said surface of the artefact (1) thereby to improve the tribological properties of the load-bearing surface of the artefact (1).

18. An artefact according to Claim 17 adapted to form a component of a prosthesis.

19. An artefact according to Claim 17 or 18 wherein the artefact is made of ultra high molecular weight poly-ethylene.

# Fig.1.

# Fig.2.

European Patent
Office

**EUROPEAN SEARCH REPORT**

Application Number

EP 95 30 0480

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DIAMOND AND RELATED MATERIALS, vol. 3, no. 3, February 1994 LAUSANNE CH, pages 205-209, A.R. MCCABE ET AL. 'MECHANICAL PROPERTIES OF ION-BEAM DEPOSITED DIAMOND-LIKE CARBON ON POLYMERS' * the whole document * | 1-9 | A61L27/00 C23C14/02 C23C14/06 |
| P,X | DIAMOND AND RELATED MATERIALS , vol. 3, no. 10, October 1994 LAUSANNE CH, pages 1265-1269, A.R. MCCABE ET AL. 'MODIFICATION OF THE ELECTRONIC PROPERTIES OF ION-BEAM-DEPOSITED DIAMOND-LIKE CARBON ON POLYMERS' * figure 1 * | 1-9 | |
| D,Y | GB-A-2 122 224 (UNITED KINGDOM ATOMIC ENERGY AUTHORITY) 11 January 1984 * claims * | 1-19 | |
| D,Y | US-A-5 133 757 (PIRAN SIOSHANSI ET AL.) 28 July 1992 * claims; examples * | 1-19 | TECHNICAL FIELDS SEARCHED (Int.Cl.6) A61L C23C |
| A | WO-A-91 16013 (SPIRE CORP.) 31 October 1991 * claims * | 1-19 | |
| A | GB-A-2 128 637 (TECHNION RESEARCH AND DEVELOPMENT FOUNDATION LTD.) 2 May 1984 * claims * | 1-19 | |

The present search report has been drawn up for all claims

| Place of search | Date of completion of the search | Examiner |
|---|---|---|
| THE HAGUE | 9 June 1995 | ESPINOSA, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)